# EUROPEAN PATENT APPLICATION

(11) **EP 1 006 179 A1**
(43) Date of publication of application: **07.06.2000**
(21) Application number: 98917704.3
(22) Date of filing: 27.04.1998
(51) Int. Cl.: C12N 15/00, C12Q 1/68

(54) **METHOD FOR DETECTING CHANGES IN GENE EXPRESSION CAUSED BY THE TRANSFER OF GENE TO BE TESTED**

(30) Priority: 28.04.1997 JP 11163597
(71) Applicant: Helix Research Institute, Chiba 292-0812 (JP)
(72) Inventor: MURAMATSU, Masaaki, Tokyo 176-0022 (JP); YANO, Kazuhiro, Kisarazu-shi Chiba 292-0804 (JP); NOGUCHI, Teruhisa, Fujisawa-shi, Kanagawa 251-0037 (JP); SUYAMA, Akira, Hachioji-shi, Tokyo 192-0372 (JP)
(74) Representative: VOSSIUS & PARTNER
(86) International application number: JP9801935
(87) International publication number: WO9849282

(57) **Abstract**

A method for detecting changes in gene expression in cells caused by the expression of a gene to be tested comprises extracting mRNAs from cells into which the gene to be tested has been transferred and expressed and control cells into which the gene to be tested has not been transferred and comparing the constitution of these mRNAs. This method also enables screening a gene whose expression is changed in cells due to the expression of the gene to be tested in the cells.

## Description

### Technical Field

The present invention relates to a method for detecting changes in gene expression caused by the transfer of a gene to be tested, and a method for screening a gene whose expression is changed due to the transfer of the gene to be tested into cells.

### Background Art

A gene does not exist independently as such in the living organism, but is closely associated with other genes, participating in regulation of the expression of other genes, as a constituent of the functional network. When one gene is isolated and its structure can be identified as well as other genes closely associated with the gene in the living organism, it will become a useful means of analyzing the function of the isolated gene. Especially, if an unknown gene is isolated and its expression can be correlated with that of a known gene, this information can be useful for the functional analysis of this unknown gene. However, there have been no reports on the method for detecting the relationship between the expression of a specific gene and that of other genes.

The method for detecting the difference in gene expression between two types of cells (or tissues) that are placed in different states have been developed, including the differential hybridization method (N. Zhao, H. Hashida, N. Takahashi & Y.Sakaki, Cloning and sequence analysis of the human SNAP25 cDNA, Gene 145: 313-314 (1994)), the differential display (DD) method (T. Ito, K. Kito, N. Adati, Y. Mitsui, H. Hagiwara & Y. Sakaki, Fluorescent differential display: arbitrarily primed RT-PCR fingerprinting on an automated DNA sequencer, FEBS Lett. 351: 231-236 (1994)), and so on. These methods have been used, for example, for detecting the difference in gene expression between cells that are at the different developmental stages or different phases of the cell cycle, between wild type cells the and their mutants deficient in a specific gene, or between normal tissues and pathological tissues.

All of these methods have been used for comparing genes expressed in specific cells without changing the constitution of the gene, and there have been no reports on the use of these methods for detecting changes in the expression of the endogenous gene due to the influence of a gene artificially transferred into cells.

### Disclosure of the Invention

An objective of the present invention is to provide a method for detecting changes in the expression of intracellular genes caused by the expression of a specific gene transferred into cells, and a method for screening a gene whose expression is changed in cells due to the transfer of a specific gene into the cells.

The present inventors studied to solve the above-described problems, and found that changes in the gene expression pattern in cells caused by the expression of a gene to be tested is able to be detected by transferring the gene to be tested into cells to allow the cells to express it, extracting intracellular mRNAs from the cells and from control cells into which the gene to be tested has not been transferred, and comparing the constitution of both mRNAs. Furthermore, this method is used to screen genes whose intracellular expression is changed due to the expression of the gene to be tested.

Moreover, the present inventors focused on the fluorometric method for detecting differences in the expression level of a specific gene in different cells (David J. Lockhart, Helin Dong, Michael C. Byrne, Maximillian T. Follettie, Michael V. Gallo, Mark S. Chee, Michael Mittmann, Chunwei Wang, Michiko Kobayashi, Heidi Horton, and Eugene L. Brown, Expression monitoring by hybridization to high-density oligonucleotide arrays, Nature Biotechnology 14:1675-1680 (1996)), and studied to apply this method to the present invention. The inventors then succeeded in conveniently and effectively detecting changes in gene expression in cells caused by the expression of the gene to be tested by labeling cDNAs obtained from each of isolated mRNAs with different fluorescent labels, mixing both labeled cDNAs, hybridizing them to a specific probe, and detecting the intensity of fluorescence emitted from cDNAs hybridized to the probe, where the fluorescence tone varies based on the difference in the existing amount of each cDNA hybridized to the probe.

The present invention relates to a method for detecting changes in gene expression caused by the transfer of a gene to be tested, and a method for screening a gene whose expression is changed due to the transfer of a gene to be tested. The invention preferably relates to a method for effectively detecting and screening a gene by determining changes in the fluorescence tone, more specifically to:
(1) a method for detecting changes in the gene expression pattern in cells caused by the expression of a gene to be tested, the method comprising comparing (a) mRNA in specific cells into which an gene to be tested that is expressible in the cells has been transferred, with (b) mRNA in specific cells into which the gene to be tested has not been transferred;
(2) a method for detecting changes in the gene expression pattern in cells caused by the expression of a gene to be tested, the method comprising comparing (a) mRNA in specific cells carrying an expression vector into which the gene to be tested has been expressibly transferred, with (b) mRNA in specific cells carrying an expression vector into which the gene to be tested has not been transferred;
(3) the method according to (1) or (2), the method further comprising reverse transcribing mRNAs of (a) and (b);
(4) the method according to (1) or (2), the method further comprising detecting changes in the expression pattern of mRNA corresponding to the specific probe DNA caused by the expression of the gene to be tested based on the difference in the amount of mRNAs in groups (a) and (b), which hybridize to the specific probe DNA;
(5) the method according to (1) or (2), the method further comprising detecting changes in the expression pattern of mRNA corresponding to the specific probe DNA caused by the expression of the gene to be tested by the difference in the amount of cDNAs obtained by reverse transcription of mRNAs of groups (a) and (b), which hybridize to the specific probe DNA;
(6) the method according to (5), the method comprising labeling cDNAs obtained by reverse transcription of mRNAs of groups (a) and (b) with different fluorescent labels, and detecting the difference in the amount of cDNAs hybridized to the probe DNA based on the intensity of fluorescence emitted from cDNAs hybridized to the probe DNA;
(7) the method according to any one of (4) to (6), the method comprising hybridizing the mRNA or the cDNA to multiple probe DNAs;
(8) the method according to (6) or (7), the method comprising labeling one group of cDNAs with rhodamine and the other group with FITC;
(9) the method according to (1) or (2), the method comprising performing RT-PCR on mRNAs of groups (a) and (b), subjecting the transcripts to gel electrophoresis, and detecting changes in the gene expression pattern in cells caused by the expression of the gene to be tested based on the difference in the electrophoretic patterns;
(10) a method for screening a gene whose expression level is changed in cells due to the expression of the gene to be tested, the method comprising comparing (a) total mRNA in specific cells into which a gene to be tested that is expressible in the cells has been transferred, with (b) total mRNA in specific cells into which the gene to be tested has not been transferred, and detecting a gene of (a) whose expression level differs from that of (b);
(11) a method for screening a gene whose expression level is changed in cells due to the expression of a gene to be tested, the method comprising comparing (a) total mRNA in specific cells carrying an expression vector into which the gene to be tested has been expressively transferred, with (b) total mRNA in specific cells carrying the expression vector into which the gene to be tested has not been transferred, and detecting a gene of (a) whose expression level differs from that of (b);
(12) the method according to (10) or (11), the method comprising detecting a gene whose intracellular expression level is increased or decreased due to the expression of the gene to be tested based on the difference in the amounts of mRNAs of groups (a) and (b), which hybridize to the specific probe DNA;
(13) the method according to (10) or (11), the method comprising detecting a gene whose intracellular expression level is increased or decreased due to the expression of the gene to be tested based on the difference in the amounts of cDNAs obtained by reverse transcribing mRNAs of groups (a) and (b), wich hybridize to the specific probe DNA;
(14) the method according to (13), the method comprising labeling each group of cDNAs obtained by reverse transcription of mRNAs of (a) and (b) with different fluorescent labels, and detecting the difference in the amount of cDNAs of each group, which hybridize to the probe DNA, based on the intensity of fluorescence emitted from cDNAs hybridized to the probe DNA;
(15) the method according to any one of (12) to (14), the method comprising hybridizing the mRNA or the cDNA to multiple probe DNAs;
(16) the method according to (14) or (15), the method comprising labeling one group of cDNAs with rhodamine and the other group with FITC;
(17) a gene whose intracellular expression level is increased or decreased due to the expression of the specific gene, wherein said gene is detectable by the method according to any one of (10) to (16);
(18) a vector comprising the gene according to (17);
(19) a transformant carrying the vector according to (18); and
(20) a protein or peptide comprising the amino acid sequence encoded by the gene according to (17).

First, the present invention relates to a method for detecting changes in the gene expression pattern in cells caused by the expression of a gene to be tested, the method comprising comparing mRNAs in cells into which a gene to be tested that is expressible in the cells has been transferred with mRNAs in cells into which the gene to be tested has not been transferred.

In the detection method of this invention, a gene to be tested, which is transferred into cells, is not particularly limited, and any desired gene can be used. The host cells into which the gene to be tested are transferred is also not particularly limited, and various animal or plant cells and microbial cells can be used. Preferable examples of animal cells include COS cells, NT-2 cells, Ba/F3 cells, and so on. Cells used in the detection method of this invention include not only cultured cells but also cells in animal and plant bodies.

The gene to be tested is preferably inserted into a suitable expression vector and then transferred into cells. The vectors are not particularly limited as long as they are compatible with host cells. For example, the "pME18S vector" is preferably used for COS cells and NT-2 cells (Y. Takebe, M. Seiki, J. Fujisawa, P. Hoy, K. Yokota, K. Arai, M. Yoshida & N. Arai, SR alpha promoter: an efficient and versatile mammalian cDNA expression system composed of the simian virus 40 early promoter and the R-U5 segment of human T-cell leukemia virus type 1 long terminal repeat, Mol. Cell Biol. 8: 466-72 (1988)). The "pCEP4 vector" (Invitrogen) is suitable for Ba/F3 cells. The gene to be tested can be transferred into cells by standard methods known to those skilled in the art, such as the lipofectamine method, electroporation, microinjection, etc.

Expressed mRNAs can be isolated from respective cells, for example, by the guanidium method or the method using an oligo-dT column (Current protocols in Molecular Biology, edit. Ausubel et al. (1987) Publish. John Wiley & Sons). In the detection method of the present invention, total mRNAs isolated from cells are usually used to detect changes in their gene expression. mRNAs fractionated by various methods can also be used depending on the experimental purpose.

In the detection method of this invention, not only mRNAs but also cDNAs obtained by reverse transcription of mRNA may be used. Reverse transcription of mRNA can be carried out by the superscript method using oligo-dT as a primer (Current protocols in Molecular Biology, edit. Ausubel et al. (1987) Publish. John Wiley & Sons), etc. In addition, when mRNAs whose expression level in cells is low, the detection sensitivity can be enhanced by subjecting it to RT-PCR (Current protocols in Molecular Biology, edit. Ausubel et al. (1987) Publish. John Wiley & Sons).

Isolated mRNAs or cDNAs can be compared, for example, based on the difference in the electrophoretic patterns obtained by subjecting them to electrophoresis. The comparisons are easily carried out by amplifying cDNA synthesized from isolated mRNA by PCR, and electrophoresing the PCR product.

The method of this invention can detect the difference in the amount of a particular mRNA or cDNA using a specific probe DNA as well as overall changes in the gene expression. First, a specific probe DNA is hybridized with total mRNAs (or cDNA group) expressed in specific cells into which the gene to be tested has been transferred (hereinafter referred to as "mRNA group to be tested" or "cDNA group to be tested") and with total mRNAs (or cDNA group) expressed in the specific cells into which the gene to be tested has not been transferred (hereinafter referred to as "control mRNA group" or "control cDNA group) The difference in the amount of mRNA (or cDNA) hybridizing to the specific probe DNA is then detected based on changes in the expression pattern of mRNA corresponding to the specific probe DNA caused by the expression of the gene to be tested.

A specific probe DNA used in this invention is not particularly limited and can be any probe DNA corresponding to the desired gene whose changes in the expression due to the transfer of the gene to be tested is to be detected. Labels for the probe DNA include, for example, radioisotope labels such as ³²P, DIG label (Boehringer Mannheim), fluorescence labels such as FITC, rhodamine, etc.

Methods for detecting mRNA (or cDNA) hybridizing to the probe DNA include, for example, Southern blotting, the differential method, the differential display method, the molecular index method, the RNA dot blot method, Northern hybridization, the primer extention method, the S1 mapping method, the quantitative PCR method, the multiplex PCR method, the DNA tip method, and so on.

In addition, the detection method of this invention can conveniently and effectively detect the difference between the amount of cDNAs hybridizing to the probe DNA in the cDNA group to be tested and that in the control cDNA group by labeling each one of cDNA groups with different fluorescent labels, mixing both labeled groups, and hybridizing them with a specific probe DNA to detect the fluorescence tone of cDNAs hybridizing to the probe DNA. For example, when one cDNA group is labeled with rhodamine, the other cDNA group with FITC, and both labeled groups are mixed and reacted with a specific probe, a yellowish fluorescence is emitted if the rhodamine-labeled cDNA and FITC-labeled cDNA, which hybridize to the specific probe DNA, are present in equal amounts, a greenish fluorescence is emitted if the FITC-labeled cDNA is present more than the other, and a reddish fluorescence is emitted if the rhodamine-labeled cDNA is present more than the other. Therefore, the difference in the amount (small or large quantity) of cDNAs hybridizing to a specific probe DNA can be detected by determining the fluorescence tone. Examples of fluorescent labels, besides rhodamine and FITC, include Texas Red, Cy2, Cy3, Cyt 5, Cy7, and so on. The detection of fluorescence can be performed using, for example, a scanning laser confocal microscope (type 1X70, Olympus).

Multiple probe DNAs can be employed in the detection method of this invention. In this case, the use of a tip formed by binding multiple probe DNAs to a base (hereinafter referred to as "genome tip") (cf. U.S. Patent 5405783) can facilitate a convenient and effective detection of changes in expression of many genes due to the transfer of the gene to be tested. In the method of detecting the fluorescence tone, for example, the cDNA group is hybridized to multiple probe DNAs immobilized on the glass base of a genome tip and the fluorescence intensity of cDNAs hybridizing to each probe is detected using a scanning laser microscope to simultaneously detect differences in the amounts of cDNAs hybridizing to multiple probe DNAs. The detection method using a genome tip is advantageous in that the procedures can be repeated readily and economically.

Second, the present invention relates to a method for screening a gene whose expression level is changed out of genes whose changes in the expression have been detected by the above-described detection method.

The screening method of the present invention enables selecting, a desired gene (e.g. a gene whose expression level is increased or decreased from a predetermined value) out of genes whose changes in the expression have been detected by the method of this invention. If the probe DNA for a known gene is used in the hybridization, the gene selected can be identified as the known gene (or a gene having a similar nucleotide sequence).

Once a gene whose expression level is changed due to the transfer of the gene to be tested into cells is isolated by the screening method of this invention, it is possible to insert the gene to an appropriate vector, transfer this vector into host cells, and produce the protein encoded by the gene from the transformant thus obtained. Examples of the host cells to which the vector is transferred include various animal cells (e.g. COS cell, etc.), *Escherichia coli*, yeasts, insect cells, and so on. As to vectors, the "pME18S vector" is preferably used for COS cell and NT-2 cell (Y. Takebe, M. Seiki, J. Fujisawa, P. Hoy, K. Yokota, K. Arai, M. Yoshida & N. Arai, SR alpha promoter: an efficient and versatile mammalian cDNA expression system composed of the simian virus 40 early promoter and the R-U5 segment of human T-cell leukemia virus type 1 long terminal repeat, Mol. Cell Biol. 8: 466-72 (1988)), etc. The "pET vector" (Takara) or the like is used for *Escherichia coli* cells and the "BacPac vector" (Clontech) for insect cells. The vector can be introduced into host cells by the standard method, for example, the electroporation method for *Escherichia coli*, the lipofectamine (GIBCO-BRL) method for COS cells, and the method using the baculovirus infection for insect cells. The recombinant protein can be purified from transformants expressing the protein by the appropriate combination of standard methods such as various chromatographies, electrophoresis, gel filtration, etc. depending on properties of the protein. In addition, column purifications using the His-tag method or the HA-tag method can be also used.

### Best Mode for Implementing the Invention

The present invention will be described in more detail with reference to the following examples, but is not to be construed as being limited thereto.

### [Example 1] Preparation of tips having DNA immobilized on base

Oligo-DNAs (Table 1) having sequences 1 to 6 (described in SEQ ID Nos: 1 to 6) were immobilized on a glass base by the optical lithograph method (Hermanson, G. T., et al., "Immobilized Affinity Ligand Techniques," Academic Press, Inc., San Diego, CA (1992); Robertson, S.A., et al., J.Am. Chem. Soc., 113, 2722-2729 (1991)). Herein, sequences 1, 2, and 3 are probes for detecting the c-fos gene (C. Van Beveren, F. van Straaten, T. Curran, R. Muller & I. H. Verma, Analysis of FBJ-MuSV provirus and c-fos (mouse) gene reveals that viral and cellular fos gene products have different carboxy termini, Cell, 32: 1241-55 (1983) [MUID: 83180421]), and sequences 4, 5, and 6 are probes for detecting β-actin (S. Alonso, A. Minty, Y. Bourlet & M. Buckingham, Comparison of three actin-coding sequences in the mouse; evolutionary relationships between the actin genes of warm-blooded vertebrates, J. Mol. Evol. 23: 11-22 (1986) [MUID: 86200234]).

**Table 1**

| | |
|---|---|
| Sequence 1 | 5' -AGCAGCAGCAACGAGCCCTCCTCCGACTCCCTGAGCTCA CCCACGCTGCTGGCCCTGTGA-3' |
| Sequence 2 | 5' -CTCCGACTCCCTGAGCTCACCCACGCTGCTGGCCCTGTGA-3' |
| Sequence 3 | 5'-CCACGCTGCTGGCCCTGTGA-3' |
| Sequence 4 | 5'-TGGCTCCATCCTGGCCTCACTGTCCACCTTCCAGCAGATGTGGAT CAGCAAGCAGGAGTA-3' |
| Sequence 5 | 5' -TGTCCACCTTCCAGCAGATGTGGATCAGCAAGCAGG AGTA-3' |
| Sequence 6 | 5' -TGGATCAGCAAGCAGGAGTA-3' |

First, the surface of a slide glass (MATSUNAMI, S0313) was treated with 0.05 M HCl for 1 h, washed with ultrapure water several times, and then soaked in ethanol. Next, the slide glass was successively soaked in the solution A (diethyl ether : toluene = 1 : 1), solution B (toluene), and solution C (toluene : 3-(2-aminoethylaminopropyl)-trimethoxysilane = 10 : 1). The container containing the slide glass soaked in the solution C was then transferred as a whole into a desiccator. The air was replaced with the nitrogen gas, and the aminosilanation reaction was allowed to proceed overnight. The slide glass was then washed with ethanol several times, further with ultrapure water for 2 h, and vacuum-dried in a desiccator (cf. Hermanson, G. T. et al., Academic Press, Inc., San Diego, California (1992)). A capping agent (4,5-dimethoxy-2-nitrobenzylchloroformate dissolved in tetrahydrofuran (THF), 10 mg/ml) was placed on the silanized slide glass, and allowed to react for 10 min. The slide glass was then washed successively with THF and ultrapure water, and dried in the nitrogen stream (Cf. Robertson, S. A., et al., J. Am. Chem. Soc., 113, 2722-2729 (1991)). On the dried slide glass to be decapped was placed 1 mM potassium acetate (pH 4.5), and decapping was performed by irradiating the glass with the excitation light of a mercury-vapor lamp (using a 330-380 filter) for 30 min under a microscope. After the reaction, the slide glass was washed several times with ultrapure water, and 10 mM DSS (disuccinimidyl suberate) was coated on the glass to allow it to react for 10 min. The glass was then washed and dried by blowing the nitrogen gas. Further, 100 µM oligo-DNA (0.2 µl) was added to the surface of the slide glass, and allowed to react for 3 min. Then, the slide glass was rinsed several times with TE (Tris-HCl (pH 7.5), EDTA 1 mM) to remove unreacted NHS groups, washed with ultrapure water, and dried in the nitrogen gas stream.

### [Example 2] Preparation of mRNA from cultured cells and synthesis of fluorescence-labeled cDNA

The expression vector "SRa-PKA" for cAMP-dependent protein kinase (hereinafter abbreviated as PKA) (M. Muramatsu, K. Kaibuchi & K. Arai, A protein kinase C cDNA without the regulatory domain is active after transfection in vivo in the absence of phorbol ester, Mol. Cell Biol. 9: 831-6 (1989) [89219080]) and the control vector "SRa" (Y. Takebe, M. Seiki, J. Fujisawa, P. Hoy, K. Yokota, K. Arai, M. Yoshida & N. Arai, SR alpha promoter: an efficient and versatile mammalian cDNA expression system composed of the simian virus 40 early promoter and the R-U5 segment of human T-cell leukemia virus type 1 long terminal repeat, Mol. Cell Biol. 8: 466-72 (1988) [88094421]) were transferred respectively into the cultured Ba/F3 cells using lipofectamine (GIBCO-BRL) (referred to as groups "a" and "b", respectively). Cells were harvested 48 h later, and mRNAs were prepared from the cells using a "Quick prep micro mRNA purification kit" (Pharmacia). After OD values of mRNAs obtained from groups "a" and "b" was measured, their concentrations were adjusted to a predetermined value, and the reverse transcriptase reaction was performed to prepare cDNAs using a "cDNA first strand kit" (Pharmacia). In this case, the cDNA prepared from the group "a" was fluorescence-labeled with "Cy5-dCTP" (Biological Detection System), while the cDNA prepared from the group "b" was fluorescence-labeled with "FluorX-dCTP" (Biological Detection System).

### [Example 3] Hybridization of fluorescence-labeled cDNA to synthetic DNA on glass base

Equal amounts of the fluorescence-labeled cDNAs prepared from samples of both groups "a" and "b" were mixed, and subjected to hybridization on the glass base using 6 x SSPE and salmon sperm DNA (0.5 mg/ml) supplemented with a mixture of the fluorescence-labeled cDNAs at 50°C for 16 h. The glass was then washed successively with 6 X SSPE (25°C) and 0.5 X SSPE (50°C).

### [Example 4] Detection of hybridized fluorescence-labeled cDNA

The fluorescence-labeled cDNAs were detected using a glass plate reader. This reader is composed of an Olympus down-illuminating fluorescence microscope combined with a holder and STI high-sensitivity camera for detection (Hamamatsu Photonics) . As a result of the measurement, a greenish interference wavelength emitted from Fluor X was observed at sites to which sequences 1, 2, and 3, c-fos gene detection probes, were bound. On the other hand, a yellowish interference wavelength emitted from Fluor X and Cy-S was seen at sites to which sequences 4, 5, and 6, β-actin detection probes, were bound. These results probed that the samples of both groups "a" and "b" contained the same amounts of β-actin mRNA, while the amounts of c-fos mRNA were significantly higher in group "a".

### Industrial Applicability

The present invention provides a method for detecting changes in the expression of genes caused by expressing a specific gene transferred into cells, and a method for screening a gene whose changes in the expression have been detected. The method of this invention utilizing the genome tip method can efficiently detect changes in the expression of many genes due to the transfer of a specific gene into cells. The method of this invention is thus extremely useful for, for example, the functional analysis of an isolated gene with unknown functions because a gene closely associated with the isolated gene can be screened.

Sequence listing

(1) Name of applicant: Helix Research Institute
(2) Title of the invention: Method for detecting changes in gene expression
caused by the transfer of gene to be tested
(3) Reference number: H1-801PCT
(4) Application number:
(5) Filing date:
(6) Country where the priority application was filed and the application number
of the application: Japan, No. Hei 9-111635
(7) Priority date: April 28, 1997
(8) Number of sequences: 6

SEQ ID NO: 1:
SEQUENCE LENGTH: 60
SEQUENCE TYPE: nucleic acid
STRANDEDNESS: single
TOPOLOGY: linear
MOLECULE TYPE: other nucleic acid, synthetic DNA
SEQUENCE DESCRIPTION: SEQ ID NO: 1
AGCAGCAGCA ACGAGCCCTC CTCCGACTCC CTGAGCTCAC CCACGCTGCT GGCCCTGTGA 60

SEQ ID NO: 2:
SEQUENCE LENGTH: 40
SEQUENCE TYPE: nucleic acid
STRANDEDNESS: single
TOPOLOGY: linear
MOLECULE TYPE: other nucleic acid, synthetic DNA
SEQUENCE DESCRIPTION: SEQ ID NO: 2
CTCCGACTCC CTGAGCTCAC CCACGCTGCT GGCCCTGTGA 40

SEQ ID NO: 3:
SEQUENCE LENGTH: 20
SEQUENCE TYPE: nucleic acid
STRANDEDNESS: single
TOPOLOGY: linear

MOLECULE TYPE: other nucleic acid, synthetic DNA
SEQUENCE DESCRIPTION: SEQ ID NO: 3
CCACGCTGCT GGCCCTGTGA 20

SEQ ID NO: 4:
SEQUENCE LENGTH: 60
SEQUENCE-TYPE: nucleic acid
STRANDEDNESS: single
TOPOLOGY: linear
MOLECULE TYPE: other nucleic acid, synthetic DNA
SEQUENCE DESCRIPTION: SEQ ID NO: 4
TGGCTCCATC CTGGCCTCAC TGTCCACCTT CCAGCAGATG TGGATCAGCA AGCAGGAGTA 60

SEQ ID NO: 5:
SEQUENCE LENGTH: 40
SEQUENCE TYPE: nucleic acid
STRANDEDNESS: single
TOPOLOGY: linear
MOLECULE TYPE: other nucleic acid, synthetic DNA
SEQUENCE DESCRIPTION: SEQ ID NO: 5
TGTCCACCTT CCAGCAGATG TGGATCAGCA AGCAGGAGTA 40

SEQ ID NO: 6:
SEQUENCE LENGTH: 20
SEQUENCE TYPE: nucleic acid
STRANDEDNESS: single
TOPOLOGY: linear
MOLECULE TYPE: other nucleic acid, synthetic DNA
SEQUENCE DESCRIPTION: SEQ ID NO: 6
TGGATCAGCA AGCAGGAGTA 20

## Claims

1. A method for detecting changes in the gene expression pattern in cells caused by the expression of a gene to be tested, the method comprising comparing (a) mRNA in specific cells into which an gene to be tested that is expressible in the cells has been transferred, with (b) mRNA in specific cells into which the gene to be tested has not been transferred.

2. A method for detecting changes in the gene expression pattern in cells caused by the expression of a gene to be tested, the method comprising comparing (a) mRNA in specific cells carrying an expression vector into which the gene to be tested has been expressibly transferred, with (b) mRNA in specific cells carrying an expression vector into which the gene to be tested has not been transferred.

3. The method according to claim 1 or 2, the method further comprising reverse transcribing mRNAs of (a) and (b).

4. The method according to claim 1 or 2, the method further comprising detecting changes in the expression pattern of mRNA corresponding to the specific probe DNA caused by the expression of the gene to be tested based on the difference in the amount of mRNAs in groups (a) and (b), which hybridize to the specific probe DNA.

5. The method according to claim 1 or 2, the method further comprising detecting changes in the expression pattern of mRNA corresponding to the specific probe DNA caused by the expression of the gene to be tested by the difference in the amount of cDNAs obtained by reverse transcription of mRNAs of groups (a) and (b), which hybridize to the specific probe DNA.

6. The method according to claim 5, the method comprising labeling cDNAs obtained by reverse transcription of mRNAs of groups (a) and (b) with different fluorescent labels, and detecting the difference in the amount of cDNAs hybridized to the probe DNA based on the intensity of fluorescence emitted from cDNAs hybridized to the probe DNA.

7. The method according to any one of claims 4 to 6, the method comprising hybridizing the mRNA or the cDNA to multiple probe DNAs.

8. The method according to claim 6 or 7, the method comprising labeling one group of cDNAs with rhodamine and the other group with FITC.

9. The method according to claim 1 or 2, the method comprising performing RT-PCR on mRNAs of groups (a) and (b), subjecting the transcripts to gel electrophoresis, and detecting changes in the gene expression pattern in cells caused by the expression of the gene to be tested based on the difference in the electrophoretic patterns.

10. A method for screening a gene whose expression level is changed in cells due to the expression of the gene to be tested, the method comprising comparing (a) total mRNA in specific cells into which a gene to be tested that is expressible in the cells has been transferred, with (b) total mRNA in specific cells into which the gene to be tested has not been transferred, and detecting a gene of (a) whose expression level differs from that of (b).

11. A method for screening a gene whose expression level is changed in cells due to the expression of a gene to be tested, the method comprising comparing (a) total mRNA in specific cells carrying an expression vector into which the gene to be tested has been expressively transferred , with (b) total mRNA in specific cells carrying the expression vector into which the gene to be tested has not been transferred, and detecting a gene of (a) whose expression level differs from that of (b).

12. The method according to claim 10 or 11, the method comprising detecting a gene whose intracellular expression level is increased or decreased due to the expression of the gene to be tested based on the difference in the amounts of mRNAs of groups (a) and (b), which hybridize to the specific probe DNA.

13. The method according to claim 10 or 11, the method comprising detecting a gene whose intracellular expression level is increased or decreased due to the expression of the gene to be tested based on the difference in the amounts of cDNAs obtained by reverse transcribing mRNAs of groups (a) and (b), wich hybridize to the specific probe DNA.

14. The method according to claim 13, the method comprising labeling each group of cDNAs obtained by reverse transcription of mRNAs of (a) and (b) with different fluorescent labels, and detecting the difference in the amount of cDNAs of each group, which hybridize to the probe DNA, based on the intensity of fluorescence emitted from cDNAs hybridized to the probe DNA.

15. The method according to any one of claims 12 to 14, the method comprising hybridizing the mRNA or the cDNA to multiple probe DNAs.

16. The method according to claim 14 or 15, the method comprising labeling one group of cDNAs with rhodamine and the other group with FITC.

17. A gene whose intracellular expression level is increased or decreased due to the expression of the specific gene, wherein said gene is detectable by the method according to any one of claims 10 to 16.

18. A vector comprising the gene according to claim 17.

19. A transformant carrying the vector according to claim 18.

20. A protein or peptide comprising the amino acid sequence encoded by the gene according to claim 17.
